Europäisches Patentamt

European Patent Office

Office européen des brevets

(11) Veröffentlichungsnummer: **0 286 956 B1**

## (12) EUROPÄISCHE PATENTSCHRIFT

(45) Veröffentlichungstag der Patentschrift: **30.12.92**

(21) Anmeldenummer: **88105438.1**

(22) Anmeldetag: **06.04.88**

(51) Int. Cl.⁵: **C12P 21/02**, C12N 15/00, C12N 1/20, C07K 13/00

(54) Gentechnologisches Verfahren zur Herstellung von Polypeptiden.

(30) Priorität: **11.04.87 DE 3712361**
**19.02.88 DE 3805150**

(43) Veröffentlichungstag der Anmeldung:
**19.10.88 Patentblatt 88/42**

(45) Bekanntmachung des Hinweises auf die
Patenterteilung:
**30.12.92 Patentblatt 92/53**

(84) Benannte Vertragsstaaten:
**AT BE CH DE ES FR GB GR IT LI LU NL SE**

(56) Entgegenhaltungen:
**EP-A- 0 001 930**
**EP-A- 0 131 868**
**EP-A- 0 281 418**
**WO-A-85/00831**

**GENE, Band 29, Nr. 1/2, July/August 1984,**
**Seiten 251-254, Elsevier Science Publishers,**
**Amsterdam, NL; L.-H. GUO et al.: "Synthesis**
**of human insulin gene. VIII. Construction of**
**expression vectors for fused proinsulin pro-**
**duction in Escherichia coli"**

(73) Patentinhaber: **HOECHST AKTIENGESELL-**
**SCHAFT**
**Postfach 80 03 20**
**W-6230 Frankfurt am Main 80(DE)**

(72) Erfinder: **Wetekam, Waldemar, Dr.**
**Zeilring 40/I**
**W-6239 Eppstein/Taunus(DE)**
Erfinder: **Jansen, Hans Willi, Dr.**
**Drosselweg 1**
**W-6230 Frankfurt am Main 80(DE)**
Erfinder: **Becker, Wolfgang**
**Lahnstrasse 27**
**W-6294 Weinbach(DE)**

**Beschreibung**

Bei der gentechnologischen Herstellung von Polypeptiden in Bakterien wird häufig das Strukturgen für das gewünschte Polypeptid im Leserahmen an das Gen für das bakterieneigene Polypeptid β-Galactosidase gekoppelt. Das Bakterium produziert dann ein Fusionsprotein, bei dem an den Carboxy-Terminus der β-Galactosidase der Amino-Terminus des gewünschten Polypeptids gebunden ist.

Bei der chemischen oder enzymatischen Abspaltung des β-Galactosidase-Anteils wird dieser in viele Bruchstücke zerlegt. Hierbei entstehen Fragmente der β-Galactosidase, die hinsichtlich der für die Auftrennung relevanten Eigenschaften wie Molgewicht ähnlich sind wie das gewünschte Protein. Hierdurch wird also die Aufarbeitung und Isolierung des gewünschten Polypeptids sehr erschwert und die Ausbeute reduziert. In Bröker, Gene Anal. Techn. 3 (1986) 53-57, ist beschrieben, wie man Fusionsproteine mit verkürzten β-Galactosidase-Anteilen herstellen kann. Als ein Vorteil dieser Fusionsproteine wird erwähnt, daß die Spaltung mit Bromcyan weniger Spaltprodukte ergibt, was die Reinigung erleichtert. Es wird hierdurch zwar die Zahl der Spaltprodukte verringert, nicht aber der vorstehend erwähnte Nachteil behoben, da auch bei diesem bekannten Verfahren Bruchstücke entstehen, die in ihren für die Auftrennung relevanten Eigenschaften dem gewünschten Produkt sehr ähnlich sind.

Erfindungsgemäß werden die Nachteile der bekannten Verfahren dadurch vermieden, daß im Gen für β-Galactosidase bzw. für ein Fragment der β-Galactosidase Codons für Methionin und/oder Arginin und/oder Cystein ganz oder teilweise durch Codons anderer Aminosäuren ersetzt werden. Hierdurch können die Bruchstücke des β-Galactosidase-Anteils so "maßgeschneidert" werden, daß die Abtrennung des gewünschten Polypeptids unproblematisch ist.

Die Erfindung bezieht sich somit auf ein Verfahren zur Herstellung eines genetisch codierbaren Polypeptids, wobei man das Strukturgen für dieses Polypeptid im korrekten Leserahmen über das Gen für die β-Galactosidase oder ein Fragment der β-Galactosidase an eine Regulationsregion koppelt, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt, das dadurch gekennzeichnet ist, daß im Gen für die β-Galactosidase bzw. für das Fragment der β-Galactosidase Codons für Methionin und/oder Arginin und/oder Cystein ganz oder teilweise durch Codons anderer Aminosäuren ersetzt sind.

Weitere Aspekte der Erfindung und ihre bevorzugten Ausgestaltungen werden im folgenden erläutert bzw. in den Patentansprüchen definiert.

Es wurde gefunden, daß Konstruktionen mit einem β-Galactosidase-Fragment mit mehr als 250 Aminosäuren, jedoch signifikant weniger als der gesamten Sequenz der β-Galactosidase, zu unlöslichen Fusionsproteinen führen, die leicht isolierbar sind. Der β-Galactosidase-Anteil weist hierbei vorteilhaft etwa 300 bis etwa 800, vorzugsweise etwa 320 bis etwa 650 Aminosäuren auf und enthält einen aminoterminalen und/oder carboxyterminalen Anteil der β-Galactosidase.

Das Gen für die β-Galactosidase bzw. das β-Galactosidase-Fragment ist im korrekten Leserahmen an eine Regulationsregion und, nötigenfalls über einen Adaptor, an das Strukturgen für das gewünschte Polypeptid gebunden.

Der Adaptor, der gegebenenfalls auch entfallen kann, kodiert hierbei vorteilhaft für eine Aminosäure (oder eine Gruppe von Aminosäuren), die vor dem Amino-Terminus des gewünschten Polypeptids liegt und dessen leichte - chemische oder enzymatische - Abtrennung vom β-Galactosidase-Anteil erlaubt. Wenn das gewünschte Polypeptid beispielsweise kein Methionin enthält bzw. gentechnologisch so variiert wurde, daß es kein Methionin enthält, so wählt man vorteilhaft als Aminosäure vor dem Amino-Terminus des gewünschten Polypeptids Methionin, worauf durch Chlorcyan- oder Bromcyan-Spaltung das gewünschte Polypeptid vom β-Galactosidase-Anteil abgetrennt werden kann. Wenn das gewünschte Polypeptid durch Trypsin nicht inaktiviert wird, dann kann vor den Amino-Terminus des gewünschten Polypeptids die Aminosäure Arginin programmiert werden, worauf das gewünschte Polypeptid durch eine Trypsinspaltung gewonnen wird. Selbstverständlich kann in diesem Falle auch eine im gewünschten Polypeptid vorhandene Trypsinspaltstelle gentechnologisch aufgehoben werden.

Verkürzte β-Galactosidase-Konstruktionen sind generell bevorzugt, da die Kapazität der Wirtszelle für die Produktion des Fremdproteins begrenzt ist und somit ein größerer Anteil des Fusionsproteins für das gewünschte Protein zur Verfügung steht. Hierdurch wird also schon die Ausbeute entscheidend verbessert. Ein weiterer Vorteil ist, daß die unlöslichen Fusionsproteine nicht nur nach dem Zellaufschluß leicht isolierbar sind, sondern auch von wirtseigenen Proteasen nicht nennenswert abgebaut werden. Somit ist eine längere Induktionsperiode möglich, was zu einer höheren Akkumulation des Fremdproteins im Bakterium führt. Darüber hinaus ergeben sich durch die erleichterte Aufarbeitung geringere Ausbeuteverluste bei der Isolierung des gewünschten Proteins, so daß insgesamt signifikant höhere Ausbeuten erhalten werden, als sie nach den bekannten Verfahren resultieren.

Die Figuren veranschaulichen die bevorzugte Ausgestaltung der Erfindung mit verkürzten $\beta$-Galactosidase-Konstruktonen, wobei nicht berücksichtgt ist, in welchem Ausmaß die "unerwünschten" Codons durch "erwünschte" ausgetauscht sind.

Fig. 1 zeigt Konstruktionen von verkürzten $\beta$-Galactosidase-Sequenzenohne (A 1. - 5.) und mit (B 1. - 5.) Linker.

Fig. 2 zeigt die Konstruktion des Plasmids pWZRI (ein Derivat des Plasmids pBR 322 mit $\beta$-Galactosidase-Fragmenten aus den Plasmiden pUC 9 und pUR 270).

Fig. 3A - 3D zeigen die Konstruktion des Plasmids pWI 6 mit der Affen-Proinsulin-DNA.

Fig. 4 zeigt die Konstruktion des Plasmids pWZIPdMdC aus pWI 6 (Fig. 3D) und dem $\beta$-Galactosidase-Fragment A 2 aus Fig. 1.

Fig. 5 zeigt die Konstruktion des Plasmids pWZPWB1dMdC aus pWI 6 (Fig. 3D) und pWZIPdMdC (Fig. 4). pWZPWB1dMdC enthält einen Polylinker (MCS), der den Einbau von Genen zur Expression der gewünschten Polypeptide ermöglicht.

Die Regulationsregion kann natürlich, insbesondere bakterieneigen, chemisch-synthetisch oder eine Hybridregion sein, beispielsweise der Fusionspromotor tac. Die Regulationsregionen enthalten zusätzlich einen Operator, z.B. den lac-Operator, und 6 bis 14 Nucleotide vor dem Methionin-Codon der $\beta$-Galactosidase-Fragmente eine ribosomale Bindungsstelle.

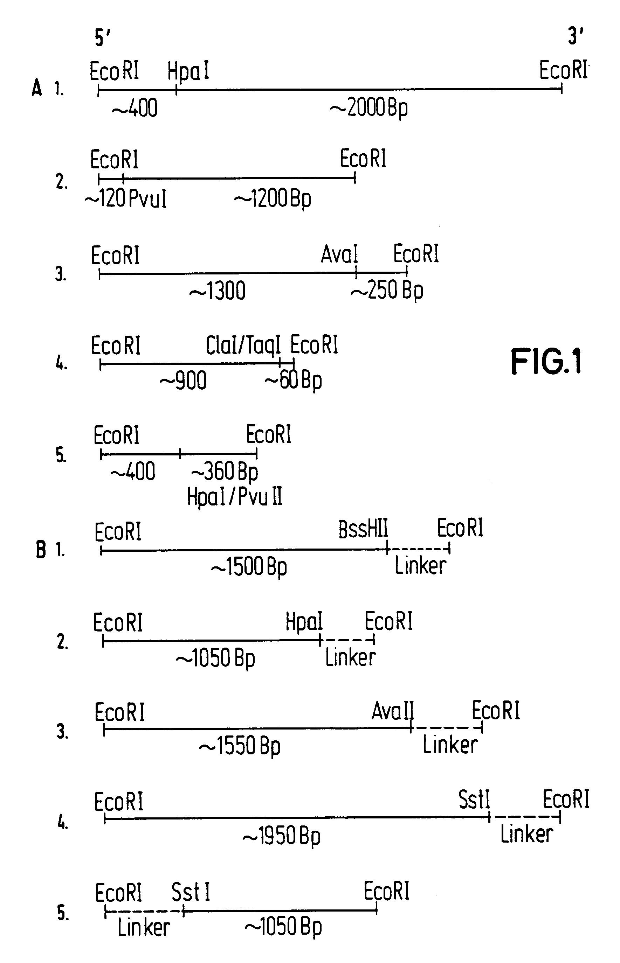

Das $\beta$-Galactosidase-Fragment besteht vorteilhaft aus einer Fusion einer aminoterminalen und carboxyterminalen Teilsequenz. Hierdurch wird der Anteil der $\beta$-Galactosidase im Fusionsprotein erheblich reduziert. Außerdem erlaubt diese Konstruktion den Austausch verschiedener Regulationssysteme ohne besonderen Aufwand. Es können jedoch auch ausschließlich aminoterminale oder überwiegend carboxyterminale Sequenzen der $\beta$-Galactosidase eingesetzt werden. Für diese Konstruktionen von $\beta$-Galactosidase-verkürzungen kann von natürlichen Restriktionsenzym-Schnittstellen Gebrauch gemacht werden. Es können aber auch Konstruktionen mit chemisch-synthetischen Linkern bzw. Adaptoren verwendet werden, die einen korrekten Leserahmen - frei von Stop-Codons - gewährleisten und gegebenenfalls ein ATG-Startcodon enthalten. Die Figur 1 A zeigt $\beta$-Galactosidase-Fragmente, bei denen von natürlichen Restriktionsenzymschnittstellen Gebrauch gemacht wurde, die Figur 1 B solche unter Verwendung eines Linkers.

Weitere Modifikationen des $\beta$-Galactosidase-Fragmentes können sich von Fall zu Fall einzeln oder in Kombination als vorteilhaft erweisen.

Bei durch Chlorcyan- oder Bromcyan-Spaltung vom $\beta$-Galactosidase-Fragment abzutrennenden Polypeptiden erleichtert es die Reinigung der letzteren, wenn man durch gezielte in-vitro-Mutagenese einige oder vorteilhaft alle Codons der störenden Methionin-Reste durch Codons für andere Aminosäuren, vorzugsweise Leucin oder Isoleucin, ersetzt.

Die Spaltung führt bei einem so modifizierten Fusionsprotein neben dem gewünschten Polypeptid zu einer verringerten Zahl an $\beta$-Galactosidase-Spaltfragmenten, die so gewählt werden können, daß sie sich aufgrund ihrer Größe und/oder Ladung leicht vom gewünschten Polypeptid trennen lassen.

Bei Polypeptiden, die durch saure Spaltung der Peptidbindung zwischen Asparaginsäure und Prolin vom $\beta$-Galactosidase-Fragment abgetrennt werden sollen, kann es von Vorteil sein, die entsprechenden Codons im $\beta$-Galactosidase-Fragment durch gezielte in-vitro-Mutagenese so zu verändern, daß an diesen Stellen keine saure Spaltung mehr möglich ist, vorzugsweise durch Umwandlung des Codons für Asparaginsäure in ein Codon für Glutaminsäure.

Bei Polypeptiden, die durch tryptische Spaltung vom $\beta$-Galactosidase-Fragment abgetrennt werden sollen, kann man durch gezielte in-vitro-Mutagenese Codons für Arginin und/oder Lysin so verändern, daß die nach tryptischer Spaltung entstehenden $\beta$-Galactosidase-Bruchstücke aufgrund ihrer Größe und/oder Ladung leicht vom gewünschten Polypeptid abgetrennt werden können.

Bei Polypeptiden, die kein Cystein enthalten, kann ein Codon für die Aminosäure Cystein zwischen die für das $\beta$-Galactosidase-Fragment und die für das gewünschte Polypeptid codierende DNA eingefügt werden. Durch nachfolgende spezifische S-Cyanylierung kann dann eine Abspaltung des gewünschten Polypeptids vom $\beta$-Galactosidaseanteil erfolgen.

Bei Polypeptiden, für deren Aktivität die Ausbildung von Disulfidbrücken von Bedeutung ist, erweist es sich als generell nützlich, die Codons für Cystein, die im $\beta$-Galactosidase-Fragment vorhanden sind, durch gezielte in-vitro-Mutagenese in Codons für andere Aminosäuren, vorzugsweise Serin, umzuwandeln, um so eine mögliche Ausbildung falscher Disulfidbrücken zwischen dem $\beta$-Galactosidase-Fragment und dem Polypeptid unmöglich zu machen.

Der Adaptor zwischen dem $\beta$-Galactosidase-Fragment und dem Strukturgen für das gewünschte Polypeptid, der in günstigen Fällen entfallen kann, kodiert - unmittelbar vor dem Amino-Terminus des gewünschten Polypeptids - für eine Aminosäure oder eine Folge von Aminosäuren, die eine leichte Abtrennung des gewünschten Polypeptids vom β-Galactosidaseanteil erlauben. Wie bereits erwähnt, kann diese Aminosäure Methionin sein, was eine einfache Bromcyan-Spaltung erlaubt, sofern das gewünschte Polypeptid kein Methionin enthält bzw. die entsprechenden Codons gentechnologisch verändert wurden. Ein Beispiel für einen solchen Adaptor weist die folgende Nucleotidsequenz auf:

$$\text{5' \quad AAT TAT GAA TTC GCA ATG}$$
$$\text{(Eco RI) \quad TA CTT AAG CGT TAC}$$

Ein Beispiel für einen Adaptor, der im Leserahmen eine Trypsin-Spaltstelle codiert, zeigt die folgende Sequenz:

$$\text{5' \quad AAT TAT GAA TTC GCA AGA}$$
$$\text{(Eco RI) \quad TA CTT AAG CGT TCT}$$

Eine zusätzliche Erleichterung der verschiedenen Abspaltungsmodalitäten - chemisch oder enzymatisch - kann durch die sterische Separierung des gewünschten Polypeptids vom β-Galactosidase-Anteil erreicht werden. Hierzu werden über einen speziellen, chemisch synthetisierten Adaptor die Codons für eine Polyaminosäure zwischen den β-Galactosidase-Anteil und das Polypeptid eingefügt. Als Aminosäuren können unter dem Aspekt der unterschiedlichen Strukturierung dieses Poly-Aminosäure-"Arms" generell alle genetisch codierbaren Aminosäuren eingesetzt werden, beispielsweise kleine ungeladene Aminosäuren wie Glycin, Alanin, Serin oder Prolin oder geladene wie Asparaginsäure und Glutaminsäure einerseits oder Lysin und Arginin andererseits. Die Poly-Aminosäurekette umfaßt zweckmäßig 5 bis 30, vorzugsweise 10 bis 24, insbesondere 15 bis 20 Aminosäuren. Je nach Wahl der Poly-Aminosäuren läßt sich eine direkte Rückfaltung des gewünschten Genproduktes im Verbund mit dem β-Galactosidase-Anteil erreichen.

Das Strukturgen für das gewünschte Polypeptid kann in an sich bekannter Weise aus natürlichen Quellen gewonnen oder chemisch synthetisiert sein. Als Beispiel für die Isolierung eines Gens aus natürlichem Material sei auf die EP-B 0 032 675 verwiesen. Vorteilhaft sind chemisch synthetisierte Gene, die auf den speziellen Codongebrauch der Wirtszelle abgestimmt sind, wie es beispielsweise in den deutschen Offenlegungsschriften 33 27 007 (Derivate des Wachstumshormon-Releasing Factor), 33 28 793 (Derivat des Sekretin), 34 09 966 (Human-γ-Interferon), 34 14 831 (Derivate des Human-γ-Interferon), 34 19 995 (Interleukin-2 und Derivate) und 34 29 430 (Hirudin-Derivate) beschrieben oder in der (nicht veröffentlichten) deutschen Offenlegungsschrift 36 32 037 (Calcitonin) vorgeschlagen wurde.

Der Einbau der Genstruktur, bestehend aus Regulationsregion, β-Galactosidasegen-Fragment, gegebenenfalls Adaptor und Strukturgen, in einen geeigneten Vektor, das Einbringen des so gewonnenen Hybridvektors in eine geeignete Wirtszelle, die Kultivierung der Wirtszellen, der Zellaufschluß, die Isolierung und Spaltung des Fusionsproteins sowie die Isolierung des gewünschten Polypeptids sind allgemein bekannt. Es kann hierzu auf die verbreiteten Lehr- und Handbücher verwiesen werden.

Bevorzugte Vektoren sind Plasmide, insbesondere die mit E. coli verträglichen Plasmide wie pBR 322, pBR 325, pUC 8 und pUC 9 sowie andere handelsübliche bzw. allgemein zugängliche Plasmide. Der bevorzugte bakterielle Wirt ist E. coli.

In den folgenden Beispielen wird die Erfindung näher erläutert.

## Beispiel 1

20 μg des handelsüblichen Plasmids pUC 9 (vgl. Vieira et al.. Gene 19 (1982) 259 - 268; The Molecular Biology Catalogue, Pharmacia P-L Biochemicals, 1984, Appendix, S. 40) werden einer Doppelverdauung mit den Restriktionsendonukleasen Eco RI und Pvu I unterzogen und ein DNA-Fragment von 123 Basenpaaren (Bp) Länge gelelektrophoretisch abgetrennt. Diese Fragment umfaßt einen Teil der aminoterminalen Codierungssequenz der β-Galactosidase.

Zur Isolierung des carboxyterminalen Anteils des β-Galactosidasegens bis zur natürlichen Eco RI-Schnittstelle werden 20 μg des Plasmids pUR 270 (Rüther und Müller-Hill, EMBO J. 2 (1983) 1791-1794) zunächst mit Eco RI verdaut und anschließend mit dem Enzym Pvu I einer Partialverdauung unterworfen. Durch Elektrophorese auf einem 5 %igen Polyacrylamidgel wird ein DNA-Fragment von 2895 Bp abgetrennt und isoliert.

Die aminoterminalen und carboxyterminalen DNA-Fragmente des $\beta$-Galactosidasegens werden im Lauf von 6 Stunden bei 16°C zusammenligiert und das Ligierungsprodukt mit Ethanol gefällt. Die gefällte und resuspendierte DNA wird mit Eco RI geschnitten und noch einmal auf einem 5 %igen Polyacrylamidgel fraktioniert. Das DNA-Fragment der Länge 3018 Bp wird durch Elektroelution aus dem Gel isoliert und in die Eco RI-Schnittstelle des Plasmids pBR 322 ligiert. Das so erhaltene Hybridplasmid wird als pWZ RI bezeichnet.

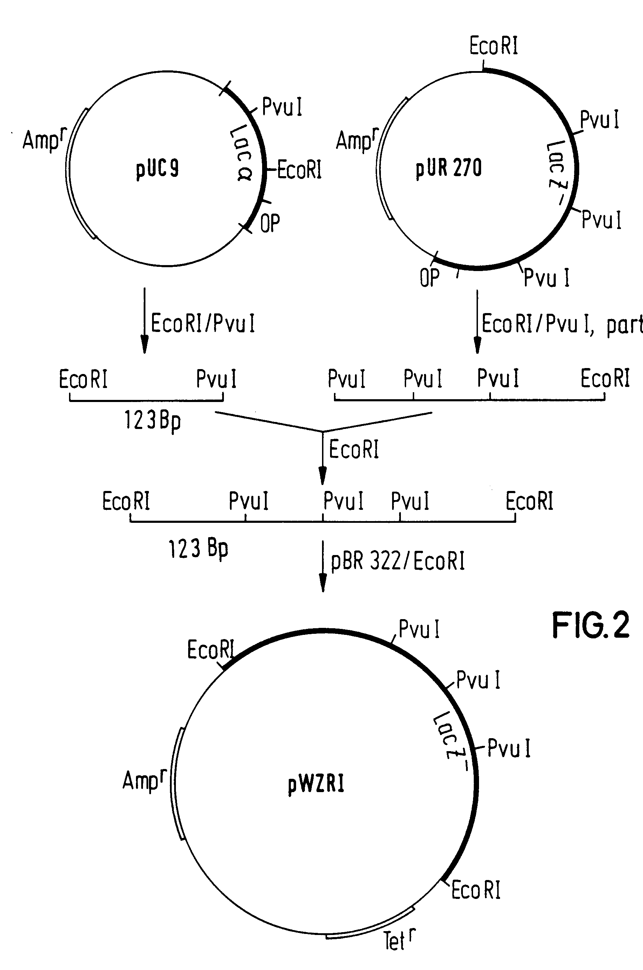

Die vorstehend beschriebenen Reaktionsschritte sind in der Figur 2 dargestellt. Die Einzelmaßnahmen wurden in bekannter Weise (Maniatis et al., Molecular Cloning, Cold Spring Harbor 1982) durchgeführt.

Das Plasmid pWZ RI wird in E. coli transformiert, dort amplifiziert und reisoliert. Durch Verdauung mit Eco RI kann das amino- und carboxyterminal verkürzte $\beta$-Galactosidase-Genfragment herausgespalten und präparativ isoliert werden. Über die bekannten Restriktionsenzym-Schnittstellen können weitere Verkürzungen konstruiert und in geeignete Expressionsplasmide eingefügt werden. Die Figur 1 A zeigt solche Verkürzungen.

Die Figur 1 B zeigt Konstruktionen mit einem chemisch-synthetischen Linker.

Sowohl die Konstruktionen aus Figur 1 A als auch die aus Figur 1 B sind so gewählt, daß der Leserahmen der verkürzten $\beta$-Galactosidase kontinuierlich übergeht in den Leserahmen des gewünschten carboxyterminalen Polypeptides. Die chemisch-synthetischen Linker der Figur 1 B können beliebig gestaltet sein, müssen jedoch selbstverständlich den Stopcodon-freien Leserahmen und gegebenenfalls ein ATG-Startcodon gewährleisten und die gewünschten Restriktionsenzym-Schnittstellen aufweisen.

**Beispiel 2**

Ein Fusionsprotein von Affenproinsulin und einer verkürzten und modifizierten $\beta$-Galactosidase ist wie folgt zugänglich:

10 μg des Plasmids pWZ RI (Fig. 2) werden mit den Restriktionsenzymen Eco RI und Pvu I geschnitten und auf einem 7,5%igen Polyacrylamidgel aufgetrennt. DNA-Fragmente von 123 und 1222 Bp Länge werden isoliert. Äquimolare Mengen der beiden DNA-Fragmente werden anschließend für 6 Stunden bei 10°C zusammenligiert und mit Eco RI nachverdaut. Das so erhaltene Ligationsgemisch wird auf einem 7,5%igen Polyacrylamidgel aufgetrennt, und die DNA-Bande mit einer Länge von 1345 Bp wird präparativ isoliert. Dieses DNA-Fragment wird in den mit Eco RI geöffneten und anschließend dephosphorylierten Vektor pBR 322 einligiert. Das so erhaltene Plasmid wird pWZP RI genannt.

Zur Entfernung der 8 in pWZP RI enthaltenen Codons für Methionin (M1-M8) und der 6 in diesem Plasmid vorhandenen Codons für Cystein (C1-C6) wird 1μg DNA von pWZP RI mit Eco RI gespalten. Das 1345 Bp große Fragment wird in den mit EcoRI geöffneten und anschließend dephosphorylierten Phagenvektor M13mp19am (Patschinsky et al., J. Virol. 59 (1986) 341-353) einligiert. Der nach Transfektion von E. coli JM101 erhaltene Phage wird als MWZPam bezeichnet. Als erster Schritt zur Entfernung der Codons für Methionin wird eine gezielte in-vitro-Mutagenese nach der "gapped duplex"-Methode (Kramer et al., Nucl. Acids Res. 12 (1984) 9441-9456) durchgeführt, wobei aufgrund der guten Effizienz dieser Methode (im Mittel 70%) die 4 Oligonucleotide dM5 - dM8 (Tab. 1) als mutagene "Primer" Verwendung finden. Die ssDNA von 12 der resultierenden Phagen wird sequenziert, wobei neben dem normalen 17mer-"Primer" auch dM7 und dC5 (Tab. 1) als"Primer" für die Sequenzierung verwendet werden. 2 der 12 DNAs weisen alle 4 gewünschten Mutationen auf, d.h. die Codons für M5-M8 sind in Codons für Isoleucin verändert worden. Diese Phagen werden als MWZP dM5,8 bezeichnet. Für die Entfernung der restlichen Methionin-Codons wird die RF-DNA von MWZP dM5,8 mit EcoRI gespalten und das 1345 Bp große DNA-Fragment in dephosphorylierten M13mp19am einkloniert. Der so erhaltene Phage wird als MWZP dM5,8am bezeichnet. Die ssDNA dieses Phagen wird einer weiteren in-vitro-Mutagenese mit dM1-dM4 als mutagenen "Primern" unterworfen. Die ssDNA von 12 der resultierenden Phagen wird sequenziert, und 3 der 12 Phagen weisen alle gewünschten Mutationen auf, d. h. die Codons für M1-M3 sind in Codons für Leucin und das Codon für M4 in ein Codon für Isoleucin verändert worden. Diese Phagen werden als MWZPdM bezeichnet. Durch Einklonieren des 1345 Bp Eco RI-Fragments aus der RF-Form dieser Phagen in den dephosphorylierten Vektor pBR 322 wird das Plasmid pWZP dM erhalten. Zur zusätzlichen Umwandlung der Codons für Cystein in Codons für Serin wird das 1345 Bp Eco RI-Fragment aus pWZP dM isoliert und in den dephosphorylierten Phagenvektor M13mp19am einkloniert. Die ssDNA des so erhaltenen Phagen MWZP dMam wird einer in-vitro-Mutagenese mit dC1-dC6 als mutagenen "Primern" unterzogen. Die ssDNA von 24 der erhaltenen Phagen wird sequenziert, wobei sich 4 der Phagenklone, die als MWZP dMdC bezeichnet werden, als die richtigen erweisen, bei denen alle Codons für Cystein in Codons für Serin umgewandelt wurden. Die RF-DNA dieser Phagen wird mit Eco RI gespalten und das 1345 Bp Eco RI-Fragment wird in den dephosphorylierten Vektor pBR 322 einkloniert, wobei man das Plasmid pWZP dMdC erhält.

Das so gewonnene, von Eco RI Schnittstellen flankierte Fragment des β-Galactosidasegens kann wie folgt in ein Plasmid integriert werden, welches eine bakterielle Regulationsregion und das Gen für Affenproinsulin über eine Eco RI-Schnittstelle miteinander verbindet:

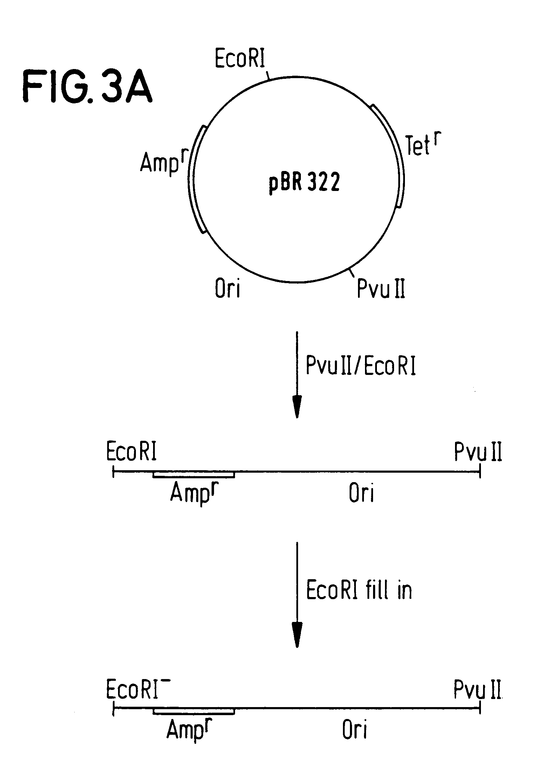

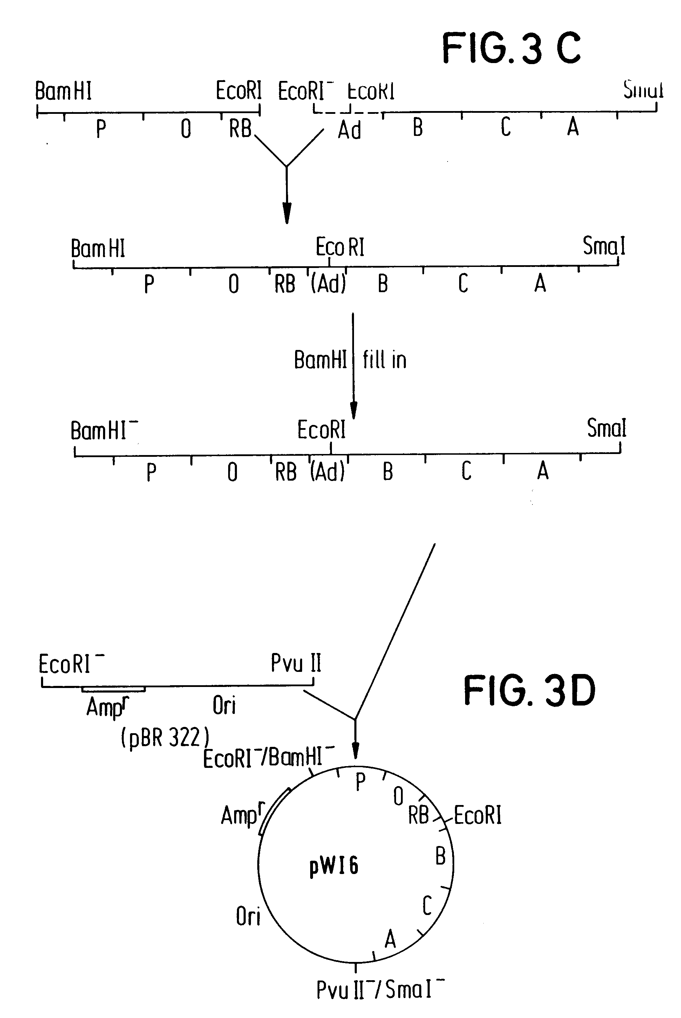

10 μg des Plasmids pBR 322 werden mit den Restriktionsendonucleasen Eco RI und Pvu II verdaut und anschließend durch eine "fill-in"-Reaktion mit Klenow-Polymerase an der Eco RI-Schnittstelle aufgefüllt. Nach gelelektrophoretischer Auftrennung in einem 5 %igen Polyacrylamidgel läßt sich das Plasmidfragment von 2293 Bp Länge durch Elektroelution gewinnen (Figur 3 A).

Aus dem Plasmid pBR 322 mit integrierter Affenpräproinsulin-DNA (Tab. 2; Wetekam et al., Gene 19 (1982) 179 - 183) wird diese durch Verdauung mit den Restriktionsendonucleasen Hind III und Fsp I isoliert und in das Plasmid pUC 9 wie folgt rekloniert: Das Plasmid pUC 9 wird mit dem Enzym Bam HI gespalten, die Spaltstelle in einer Standard-"fill-in"-Reaktion mit Klenow-Polymerase ("large fragment"), aufgefüllt, mit dem Restriktionsenzym Hind III nachgeschnitten und die DNA gelelektrophoretisch in einem 5 %igen Polyacrylamidgel von den übrigen DNA-Fragmenten abgetrennt. In das geöffnete Plasmid konnte das isolierte Insulin-DNA-Fragment von etwa 1250 Bp Länge integriert werden. Zur Abtrennung der "untranslated region" und der Präsequenz wird mit Hae III verdaut und das 143 Bp lange Fragment zur Abspaltung der letzten beiden Nucleotide aus der Präsequenz unter limitierenden Enzymbedingungen mit Bal 31 verdaut. Man erhält so am Amino-Terminus als erstes Codon TTT, das für Phenylalanin als erste Aminosäure der B-Kette steht.

An dieses Fragment wird nun ein für Eco RI spezifischer Adaptor in einer "blunt-end" Ligationsreaktion ligiert:

```
a) 5'      AAT TAT GAA TTC GCA GGA GGC GGG GGT GGC GGT GGG
(Eco RI)       TA CTT AAG CGT CCT CCG CCC CCA CCG CCA CCC


           GGC GGA GGT GGT GGC GGT GGA GGC GGT GGA GGC GGG
           CCG CCT CCA CCA CCG CCA CCT CCG CCA CCT CCG CCC


           GGT ATG
           CCA TAC


b) 5'      AAT TAT GAA TTC GCA GGA GGC GGG GGT GGC GGT GGG
(Eco RI)       TA CTT AAG CGT CCT CCG CCC CCA CCG CCA CCC


           GGC GGA GGT GGT GGC GGT GGA GGC GGT GGA GGC GGG
           CCG CCT CCA CCA CCG CCA CCT CCG CCA CCT CCG CCC


           GGT AGA
           CCA TCT
```

Um eine Polymerisierung der Adaptoren zu vermeiden, wurden diese unphosphoryliert in die Ligationsreaktion eingesetzt. Der Adaptor a) weist am Ende ein Codon für Methionin auf, der Adaptor b) das Codon für Arginin. Nach der Variante a) wird somit ein Genprodukt erhalten, bei dem durch Bromcyanspaltung eine Abtrennung des bakteriellen Anteils möglich ist, während die Variante b) eine Trypsinspaltung erlaubt.

Das Ligationsprodukt wird mit Mbo II verdaut. Nach gelelektrophoretischer Auftrennung erhält man ein DNA-Fragment von 139 Bp Länge mit der der Information für die Aminosäuren Nr. 1 bis 21 der B-Kette.

Das Gen für die restliche Information des Proinsulinmoleküls (einschließlich einer G-C-Sequenz aus der Klonierung und 21 Bp aus dem pBR 322 im Anschluß an das Stop-Codon) erhält man aus dem pUC 9-Plasmid mit der kompletten Präproaffeninsulin-Information durch Verdauung mit Mbo II/Sma I und Isolierung eines DNA-Fragments von etwa 240 Bp Länge. Durch Ligieren der beiden Proinsulin-Fragmente erhält man das korrekte Ligationsprodukt von etwa 380 Bp Länge (inklusive des Adaptors von 78 Bp). Dieses so konstruierte Proinsulin-DNA-Fragment kann nun mit einer Regulationsregion über die Eco RI-negative Schnittstelle zusammenligiert werden.

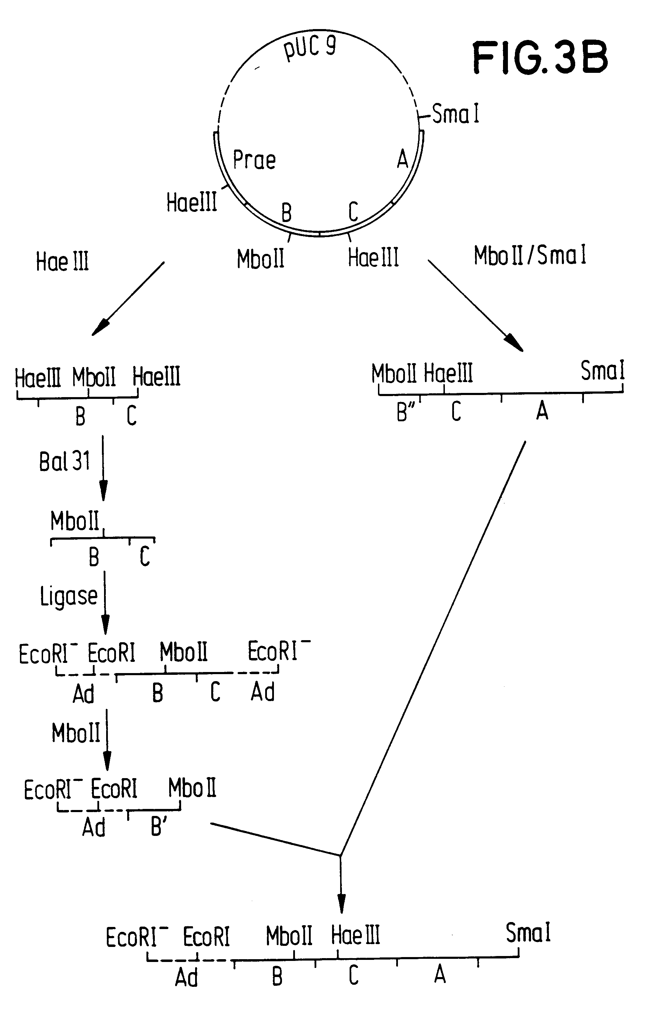

Die gesamte Reaktionsfolge zeigt die Fig. 3 B, in der A, B und C die DNA für die jeweiligen Peptidketten des Proinsulinmoleküls, Ad den (dephosphorylierten) Adaptor (a oder b) und Prae die DNA für die Präsequenz des Affenpräproinsulins bedeuten.

## Beispiel 3

Eine chemisch-synthetische Regulationsregion, bestehend aus einer Erkennungssequenz für Bam HI, dem lac-Operator, einem bakteriellen Promotor und einer ribosomalen Bindungsstelle (RB) mit einem ATG-Start-Codon, 6 bis 14 Nucleotide von der RB entfernt, und einer anschließenden Erkennungssequenz für Eco RI (Figur 3 C) wird über die gemeinsame Eco RI-Überlappungsregion mit dem nach dem vorstehenden Beispiel erhaltenen Proinsulingen-Fragment ligiert. Nach einer Doppelverdauung mit Sma I/Bam HI und einer "fill-in" Reaktion der Bam HI Schnittstelle mit dem Klenow-Fragment wird das Ligierungsprodukt (ungefähr 480 Bp) gelelektrophoretisch isoliert.

Das so erhaltene Fragment läßt sich anschließend über eine "blunt-end" Ligierung in das pBR 322-Teilplasmid gemäß Figur 3 A hineinligieren (Figur 3 D). Man erhält das Hybridplasmid pWI 6.

Nach Transformation in den E. coli-Stamm HB 101 und Selektion auf Ampicillin-Platten wurde die Plasmid-DNA individueller Klone auf die Integration eines 480 Bp-Fragments mit der Regulationsregion und dem Bal 31-verkürzten Proinsulingen getestet. Zum Nachweis der korrekten Verkürzung des Proinsulingens durch Bal 31 (Figur 3 B) wurden die Plasmide mit dem integrierten Proinsulinfragment von der Eco RI-Schnittstelle aus sequenziert. Von 60 sequenzierten Klonen hatten drei die gewünschte Verkürzung von zwei Nucleotiden (Figur 3 D).

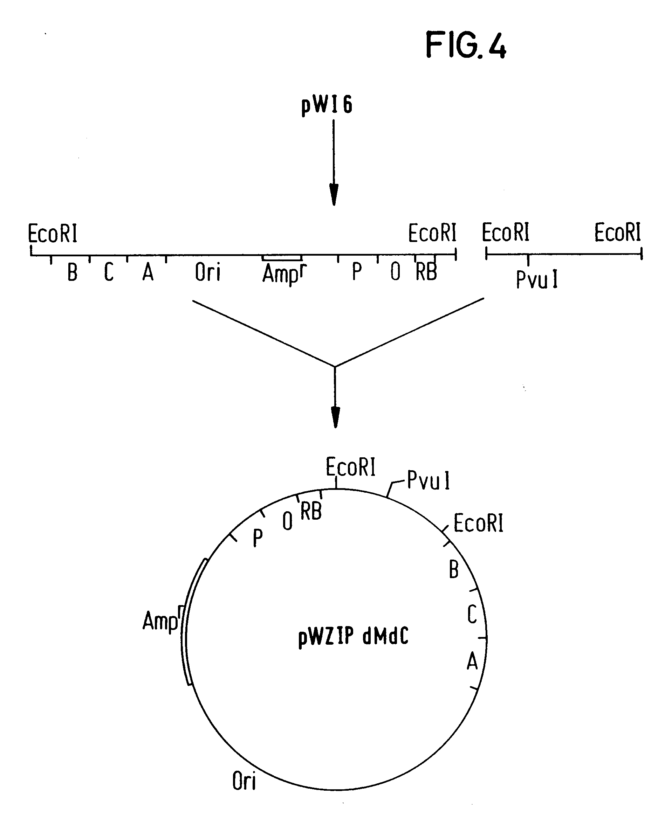

Das Hybridplasmid pWI6 dient nun als Ausgangsmaterial zur Integration der $\beta$-Galactosidasegen-Fragmente, die in Figur 1A und B wiedergegeben sind. Diese Reaktion wird hier beispielhaft durch Klonierung der in Figur 1A unter 2. aufgeführten $\beta$-Galactosidasegenverkürzung, in der zusätzlich sämtliche Codons für Methionin und Cystein durch Codons für andere Aminosäuren ersetzt wurden, vorgestellt:
Äquimolare Mengen der verkürzten und modifizierten $\beta$-Galactosidase-Gensequenz (bestehend aus etwa 120 Bp des aminoterminalen Bereichs und 1220 Bp des carboxyterminalen Bereichs, flankiert von 2 Eco RI-Schnittstellen) und des mit Eco RI gespaltenen Hybridplasmids pWI 6 (Figur 3 D) werden ligiert (Figur 4). Nach Transformation in Indikatorbakterien mit einer $\beta$-Galactosidase $\Delta$ M15-Deletion (The Lactose Operon, Ed. J. Beckwith and D. Zipser, Cold Spring Habor, 1970) reagieren nur diejenigen Bakterienkolonien auf den Indikatorfarbstoff X-gal (5-Brom-4-chlor-3-indolyl-$\beta$-D-galactosid) durch Blaufärbung, die das $\beta$-Galactosidasegen-Fragment in korrekter Richtung integriert haben. Eine eventuelle Integration mehrerer $\beta$-Galactosidasegenverkürzungen in ein Plasmid läßt sich durch eine Standardanalyse mit Restriktionsenzymen nachweisen.

Das so erhaltene Plasmid pWZIP dMdC wurde Induktion mit IPTG (Isopropyl-$\beta$-D-thiogalacto-pyranosid), dem Induktor für $\beta$-Galactosidasesynthese, auf seine Kapazitität hin getestet, ein Fusionsprodukt aus $\beta$-Galactosidase-Protein und Proinsulin zu bilden. Der Anteil dieses Produkts am Gesamtzellprotein beträgt etwa 15 bis 20 %. Das Produkt ist unlöslich und läßt sich durch Zentrifugieren leicht von den übrigen Zellbestandteilen und Proteinen abtrennen.

Die übrigen in Figur 1 A und B aufgeführten $\beta$-Galactosidasegenverkürzungen können in der gleichen Weise in das Plasmid pWI 6 (Figur 3 D) integriert werden und zeigen in E. coli ähnliche Synthesekapazitäten, wobei unlösliche Fusionsproteine erhalten werden.

Je nach Wahl des Adaptors a oder b wird das Proinsulin entweder mit Bromcyan aus dem Fusionsprotein abgespalten oder aber nach Verdauung mit Trypsin das Insulin-Derivat B 31 Arg gewonnen, aus dem das Arginin durch enzymatische Spaltung mit Carboxypeptidase B entfernt wird. Das so freigesetzte Proinsulin bzw. Insulin kann nach Standardmethoden gereinigt werden.

## Beispiel 4

Plasmide, wie pWI 6, die als Ausgangskonstruktion für die Herstellung von Fusionsexpressionsplasmiden mit Proinsulin und $\beta$-Galaktosidasegenfragmenten herangezogen werden, können vorteilhaft auch zur Expression von anderen Genprodukten brauchbar gemacht werden. Hierzu schneidet man 10 $\mu$g des

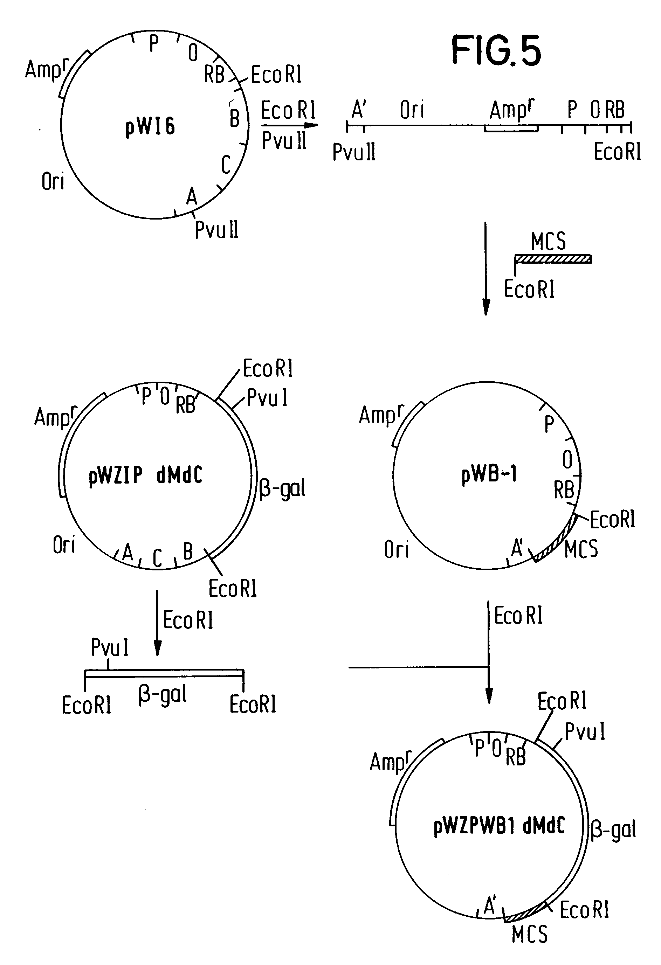

Plasmides pWl 6 mit den Restriktionsenzymen Eco RI und Pvu II (Fig. 5). Auf einem 5% Polyacrylamidgel trennt man das geöffnete, verkürzte Plasmid von den Proinsulin-Genfragmenten (324 Bp Länge) ab. Nach präparativer Isolierung des Plasmidfragmentes aus dem Polyacrylamidgel kann in ein derart geöffnetes Plasmid eine chemisch-synthetische DNA-Sequenz hineinligiert werden, die außer der Pst I-Schnittstelle eine Vielzahl von singulären Restriktionsenzym-Schnittstellen aufweist:

```
     (Eco RI)    SstI      SmaI    BamHI    XbaI      SalI      PstI
     3'  AA TTC GAG CTC GCC CGG GGA TCC TCT AGA GTC GAC CTG CAG
     5'         G CTC GAG CGG GCC CGT AGG AGA TCT CAG CTG GAC GTC


         HindIII NruI AvaIII   BglII        NcoI  SphI
     3' CCC AAG CTT CGC GAT GCA TCA GAT CTA CCA TGG CAT GCC
     5' GGG TTC GAA GCG CTA CGT AGT CTA GAT GGT ACC GTA CGG
```

Auf diese Weise erhält man das Plasmid pWB-1 (Fig. 5), welches neben einer Regulationsregion auch noch eine DNA-Sequenz enthält, die mit ihren multiplen, singulären Restriktionsenzymschnittstellen zur Klonierung von verschiedenen Genen natürlichen und chemisch-synthetischen Ursprungs geeignet ist. In Analogie zum Beispiel 2 kann jedem Gen ein chemisch synthetisierter Adaptor für eine Poly-Aminosäuresequenz und mit einer passenden Spaltstelle vorgeschaltet werden. Zwischen der Regulationsregion und der multiplen Klonierungsstelle (MCS) befindet sich eine singuläre Restriktionsenzymschnittstelle für Eco RI, in die in Analogie zu Beispiel 3 jede β-Galactosidase-Genverkürzung mit den verschiedenen Mutationsmodalitäten hineinligiert werden kann.

2 μg DNA des Plasmids pWB-1 werden mit Eco RI gespalten und anschließend dephosphoryliert. In den so behandelten Vektor wird das aus pWZIPdMdC durch Spaltung mit Eco RI gewonnene verkürzte und modifizierte β-Galactosidase-Genfragment einligiert. Nach Transformation von E. coli-Zellen und Selektion auf Expression erhält man das Plasmid pWZPWB1 dMdC, das hinter dem verkürzten und modifizierten β-Galactosidasegen die oben beschriebene multiple Klonierungsstelle enthält (Fig. 5).

**Beispiel 5**

Als Beispiel für den Einbau eines chemisch-synthetischen Gens wird im folgenden die Herstellung eines Fusionsproteins mit der Aminosäuresequenz des Lachs-Calcitonins, dem ein carboxyterminales Glycin angehängt wird, beschrieben.

Die chemisch-synthetische DNA-Sequenz für Calcitonin gemäß Tabelle 3, deren Nucleotidfolge speziell auf den Codon-Gebrauch von E. coli abgestimmt ist und die am 5′-Ende einen Bam HI-"Überhang" und vor dem Codon für die erste Aminosäure einen Adaptor, codierend für 15 Glycinreste, und ein Methionin-Codon sowie am 3′-Ende nach dem Prolin-Codon ein Glycin-Codon, zwei Stop-Codons und einen Sph I-"Überhang" aufweist, wird in das mit den Restriktionsenzymen Bam HI und Sph I geöffnete Plasmid pWZPWB1 dMdC einkloniert. Man erhält das Plasmid pWZP dMdC Calc.

Dieses Plasmid kann in bekannter Weise in E. coli transformiert, dort das entsprechende Fusionsprotein exprimiert und analog dem Proinsulin-Fusionsprotein isoliert werden. Die Abspaltung des Galactosidase-Anteils gelingt mit Bromcyan, die anschließende Reinigung nach bekannten Methoden.

## Tabelle 1

Mutagene "Primer" für die Umwandlung von Codons für
Methionin in Codons für Leucin bzw. Isoleucin und für die
Umwandlung von Codons für Cystein in Codons für Serin:

```
dM1 5'-   AG ACC GTT CAG ACA GAA CTG G
dM2 5'-   AG CGC CAC CAG CCA GTG CAG G
dM3 5'- GCA AAA ATC CAG TTC GCT GGT G
dM4 5'-    C GCC AAT CCA TAT CTG TGA AA
dM5 5'-    C GGT AAT CGC AAT TTG ACC AC
dM6 5'-    A CGG GGT ATA GAT GTC TGA CA
```

```
dM7 5'-    G GCT GGT TTC AAT CAG TTG CT
dM8 5'-    C ACC AAT CCC TAT ATG GAA ACC
dC1 5'-    G ACC GTT CAG AGA GAA CTG GCG
dC2 5'-      CAG CTC GAT GGA AAA ATC CAG TTC
dC3 5'-      ATC TGC CGT GGA CTG CAA CAA
dC4 5'-      CGC CAG CTG GGA GTT CAG GCC
dC5 5'-      GCG CTC AAA AGA GGC GGC AGT
dC6 5'-      GCG CGT CCC GGA GCG CAG ACC
```

9

**Tabelle 2**

```
                                           5' AAT TCT GCA AGA
                                           3'      GA CGT TCT
                                             (Asn)Ser Ala Arg
                                             (EcoRI)


B 1
TTT GTG AAC CAG CAC CTG TGC GGC TCC CAC CTA GTG GAA GCT CTC
AAA CAC TTG GTC GTG GAC ACG CCG AGG GTG GAT CAC CTT CGA GAG
Phe Val Asn Gln His Leu Cys Gly Ser His Leu Val Glu Ala Leu
                        Fnu4H                        AluI


TAC CTG GTG TGC GGG GAG CGA GGC TTC TTC TAC ACA CCC AAG ACC
ATG GAC CAC ACG CCC CTC GCT CCG AAG AAG ATG TGT GGG TTC TGG
Tyr Leu Val Cys Gly Glu Arg Gly Phe Phe Tyr Thr Pro Lys Thr
    BstNI                              MboII

C 1
CGC CGG GAG GCA GAG GAC CCT CAG GTG GGG CAG GTG GAG CTG GGC
GCG GCC CTC CGT CTC CTG GGA GTC CAC CCC GTC CAC CTC GAC CCG
Arg Arg Glu Ala Glu Asp Pro Gln Val Gly Gln Val Glu Leu Gly
    HpaII              AvaII DdeI                    AluI


GGG GGC CCT GGC GCA GGC AGC CTG CAG CCC TTG GCG CTG GAG GGG
CCC CCG GGA CCG CGT CCG TCG GAC GTC GGG AAC CGC GAC CTC CCC
Gly Gly Pro Gly Ala Gly Ser Leu Gln Pro Leu Ala Leu Glu Gly
    HaeIIIBstNIHhaI   Fnu4HI PstIFnu4HI     HhaI         AvaII


                   A 1
TCC CTG CAG AAG CGC GGC ATC GTG GAG CAG TGC TGC ACC AGC ATC
AGG GAC GTC TTC GCG CCG TAG CAC CTC GTC ACG ACG TGG TCG TAG
Ser Leu Gln Lys Arg Gly Ile Val Glu Gln Cys Cys Thr Ser Ile
    PstI        HhaI                    Fnu4HI


TGC TCC CTC TAC CAG CTG GAG AAC TAC TGC AAC TAA TAG TCG ACC
ACG AGG GAG ATG GTC GAC CTC TTG ATG ACG TTG ATT ATC AGC TGG
Cys Ser Leu Tyr Gln Leu Glu Asn Tyr Cys Asn
                PvuII                                SalI

TGC AGC CA        3'
ACG TCG GTT CGA   5'
PstI       (HindIII)
```

B 1, C 1 und A 1 bezeichnen den Beginn der B-, C- und A-
Kette des Affenproinsulins

10

## Tabelle 3

| Triplett-Nr. | | | | 0 | 1 | 2 | 3 |
|---|---|---|---|---|---|---|---|
| Aminosäure | | | | Met | Cys | Ser | Asn |
| Nukleotid-Nr. | | 1 | 5 | 10 | | 15 | |
| Codierender Strang | 5' | GA | TCC | ATG | TGC | TCT | AAC |
| nicht codierender Strang | 3' | | G | TAC | ACG | AGA | TTG |

| 4 | 5 | 6 | 7 | 8 | 9 | 10 | 11 | 12 | 13 |
|---|---|---|---|---|---|---|---|---|---|
| Leu | Ser | Thr | Cys | Val | Leu | Gly | Lys | Leu | Ser |
| 20 | | 25 | | 30 | 35 | | 40 | | 45 |
| CTF | TCG | ACT | TGC | GTT | CTT | GGT | AAG | CTT | TCT |
| GAC | AGC | TGA | ACG | CAA | GAA | CCA | TTC | GAA | AGA |

| 14 | 15 | 16 | 17 | 18 | 19 | 20 | 21 | 22 | 23 |
|---|---|---|---|---|---|---|---|---|---|
| Gln | Glu | Leu | His | Lys | Leu | Gln | Thr | Tyr | Pro |
| 50 | | 55 | | 60 | 65 | | 70 | | 75 |
| CAG | GAA | CTT | CAT | AAA | CTG | CAG | ACC | TAT | CCG |
| GTC | CTT | GAA | GTA | TTT | GAC | GTC | TGG | ATA | GGC |

| 24 | 25 | 26 | 27 | 28 | 29 | 30 | 31 | 32 | 33 |
|---|---|---|---|---|---|---|---|---|---|
| Arg | Thr | Asn | Thr | Gly | Ser | Gly | Thr | Pro | Gly |
| 80 | | 85 | | 90 | 95 | | 100 | | 105 |
| CGC | ACT | AAT | ACC | GGC | TCT | GGT | ACC | CCT | GGT |
| GCG | TGA | TTA | TGG | CCG | AGA | CCA | TGG | GGA | CCA |

| 34 | 35 |
|---|---|
| Stp | Stp |
| 110 | 115 |
| TAA TAG CAT G | 3' |
| ATT ATC | 5' |

**Patentansprüche**
**Patentansprüche für folgende Vertragsstaaten : AT, BE, CH, DE, FR, GB, GR, IT, LI, LU, NL, SE**

1. Verfahren zur Herstellung eines genetisch codierbaren Polypeptids, wobei man das Strukturgen für dieses Polypeptid im korrekten Leserahmen über das Gen für die $\beta$-Galactosidase oder für ein Fragment der $\beta$-Galactosidase an eine Regulationsregion koppelt, dieses $\beta$-Galactosidase-Fragment für über 250 Aminosäuren, jedoch signifikant weniger als die gesamte $\beta$-Galactosidase-Sequenz kodiert, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt, dadurch gekennzeichnet, daß im Gen für die $\beta$-Galactosidase bzw. für das Fragment der $\beta$-Galactosidase Codons für Methionin und/oder Arginin und/oder Cystein ganz oder teilweise durch

EP 0 286 956 B1

Codons anderer Aminosäuren ersetzt sind.

**2.** Verfahren nach Anspruch 1, dadurch gekennzeichnet, daß das $\beta$-Galactosidase-Fragment aus einer Fusion einer aminoterminalen und carboxyterminalen Teilsequenz besteht.

**3.** Verfahren nach Anspruch 1 oder 2, dadurch gekennzeichnet, daß das $\beta$-Galactosidase-Fragment etwa 300 bis etwa 800 Aminosäuren aufweist.

**4.** Verfahren nach einem oder mehreren der vorhergehenden Ansprüche, dadurch gekennzeichnet, daß das $\beta$-Galactosidase-Fragment etwa 320 bis etwa 650 Aminosäuren aufweist.

**5.** Verfahren nach Anspruch 1, dadurch gekennzeichnet, daß unmittelbar vor dem aminoterminalen Ende des Strukturgens ein Codon für eine Aminosäure steht, die eine chemische oder enzymatische Abtrennung des Polypeptids vom $\beta$-Galactosidase-Anteil erlaubt.

**6.** Verfahren nach einem oder mehreren der vorhergehenden Ansprüche, dadurch gekennzeichnet, daß das genetisch codierbare Polypeptid ein Derivat des Wachstumshormon-Releasing Factor, ein Interferon, ein Proinsulin, Secretin, Interleukin-2, ein Calcitonin oder ein Hirudin ist.

**7.** Genstruktur, enthaltend eine Regulationsregion, ein Gen für $\beta$-Galactosidase oder ein $\beta$-Galactosidase-Fragment,dieses $\beta$-Galactosidase-Fragment für über 250 Aminosäuren, jedoch signifikant weniger als die gesamte $\beta$-Galactosidase-Sequenz kodiert, in der $\beta$-Galactosidase-Sequenz Codons für Methionin und/oder Arginin und/oder Cystin ganz oder teilweise durch Codons anderer Aminosäuren ersetzt sind, und ein Strukturgen für ein genetisch codierbares Polypeptid, das gegebenenfalls über einen Adaptor an das Gen für die modifizierte $\beta$-Galactosidase oder das $\beta$-Galactosidase-Fragment gekoppelt ist, der den korrekten Leserahmen gewährleistet.

**8.** Vektor, enthaltend eine Genstruktur nach Anspruch 7.

**9.** Bakterium, vorzugsweise E. coli, enthaltend einen Vektor nach Anspruch, 8.

**10.** Fusionsprotein, enthaltend modifizierte $\beta$-Galactosidase oder ein $\beta$-Galactosidase-Fragment wie in einem oder mehreren der Ansprüche 1 bis 8 definiert und ein eukaryotisches genetisch codierbares Polypeptid, vorzugsweise ein Derivat des Wachstumshormon Releasing Factor, ein Interferon, ein Proinsulin, Secretin, Interleukin-2, ein Calcitonin oder ein Hirudin ist.

**Patentansprüche für folgenden Vertragsstaat : ES**

**1.** Verfahren zur Herstellung eines genetisch codierbaren Polypeptids, wobei man das Strukturgen für dieses Polypeptid im korrekten Leserahmen über das Gen für die $\beta$-Galactosidase oder für ein Fragment der $\beta$-Galactosidase an eine Regulationsregion koppelt, dieses $\beta$-Galactosidase-Fragment für über 250 Aminosäuren, jedoch signifikant weniger als die gesamte $\beta$-Galactosidase-Sequenz kodiert, diese Genstruktur in ein Bakterium einbringt, darin ein unlösliches Fusionsprotein exprimiert, dieses nach einem Zellaufschluß isoliert und das gewünschte Polypeptid durch chemische oder enzymatische Spaltung gewinnt, dadurch gekennzeichnet, daß im Gen für die $\beta$-Galactosidase bzw. für das Fragment der $\beta$-Galactosidase Codons für Methionin und/oder Arginin und/oder Cystein ganz oder teilweise durch Codons anderer Aminosäuren ersetzt sind.

**2.** Verfahren nach Anspruch 1, dadurch gekennzeichnet, daß das $\beta$-Galactosidase-Fragment aus einer Fusion einer aminoterminalen und carboxyterminalen Teilsequenz besteht.

**3.** Verfahren nach Anspruch 1 oder 2, dadurch gekennzeichnet, daß das $\beta$-Galactosidase-Fragment etwa 300 bis etwa 800 Aminosäuren aufweist.

**4.** Verfahren nach einem oder mehreren der vorhergehenden Ansprüche, dadurch gekennzeichnet, daß das $\beta$-Galactosidase-Fragment etwa 320 bis etwa 650 Aminosäuren aufweist.

**5.** Verfahren nach einem oder mehreren der vorhergehenden Ansprüche, dadurch gekennzeichnet, daß

12

EP 0 286 956 B1

das Gen für das β-Galactosidase-Fragment einer DNA-Sequenz gemäß Figur 1 entspricht.

6. Verfahren nach einem oder mehreren der vorhergehenden Ansprüche, dadurch gekennzeichnet, daß das Strukturgen für das genetisch codierbare Polypeptid über einen Adaptor an das Gen für die modifizierte β-Galactosidase oder das β-Galactosidase-Fragment gekoppelt ist.

7. Verfahren nach Anspruch 6, dadurch gekennzeichnet, daß der Adaptor für eine Polyaminosäuresequenz codiert.

8. Verfahren nach einem oder mehreren der vorhergehenden Ansprüchen dadurch gekennzeichnet, daß unmittelbar vor dem aminoterminalen Ende des Strukturgens ein Codon für eine Aminosäure steht, die eine chemische oder enzymatische Abtrennung des Polypeptids vom β-Galactosidase-Anteil erlaubt.

9. Verfahren nach einem oder mehreren der vorhergehenden Ansprüche, dadurch gekennzeichnet, daß das genetisch codierbare Polypeptid ein Derivat des Wachstumshormon-Releasing Factor, ein Interferon, ein Proinsulin, Secretin, Interleukin-2, ein Calcitonin oder ein Hirudin ist.

**Claims**
**Claims for the following Contracting States : AT, BE, CH, DE, FR, GB, GR, IT, LI, LU, NL, SE**

1. A process for the preparation of a genetically codable polypeptide, with coupling of the structural gene for this polypeptide in the correct reading frame via the gene for β-galactosidase, or for a fragment of β-galactosidase, to a regulator region, said β-galactosidase fragment coding for more than 250 amino acids, but significantly less than the complete β-galactosidase sequence, introduction of this gene structure into a bacterium, expression therein of an insoluble fusion protein, isolation thereof after cell disruption, and obtaining the desired polypeptide by chemical or enzymatic cleavage, which comprises codons for methionine and/or arginine and/or cysteine in the gene for β-galactosidase, or for the fragment of β-galactosidase, being replaced, in whole or in part, by codons of other amino acids.

2. The process as claimed in claim 1, wherein the β-galactosidase fragment is composed of a fusion of an amino-terminal and carboxyl-terminal part-sequence.

3. The process as claimed in claim 1 or 2, wherein the β-galactosidase fragment has about 300 to about 800 amino acids.

4. The process as claimed in one or more of the preceding claims, wherein the β-galactosidase fragment has about 320 to about 650 amino acids.

5. The process as claimed in claim 1, wherein a codon for an amino acid which allows chemical or enzymatic separation of the polypeptide from the β-galactosidase portion is located immediately upstream of the amino-terminal end of the structural gene.

6. The process as claimed in one or more of the preceding claims, wherein the genetically codable polypeptide is a derivative of growth hormone releasing factor, an interferon, a proinsulin, secretin, interleukin-2, a calcitonin or a hirudin.

7. A gene structure containing a regulator region, a gene for β-galactosidase or a β-galactosidase fragment, said β-galactosidase coding for more than 250 amino acids, but significantly less than the complete β-galactosidase sequence, in which β-galactosidase sequence codons for methionine and/or arginine and/or cysteine have been replaced, in whole or in part, by codons of other amino acids, and a structural gene for a genetically codable polypeptide, which is coupled where appropriate via an adaptor, which ensures the correct reading frame, to the gene for the modified β-galactosidase or the β-galactosidase fragment.

8. A vector containing a gene structure as claimed in claim 7.

9. A bacterium, preferably E. coli, containing a vector as claimed in claim 8.

13

**10.** A fusion protein containing modified $\beta$-galactosidase or a $\beta$-galactosidase fragment as defined in one or more of claims 1 to 8, and a eukaryotic genetically codable polypeptide, preferably a derivative of growth hormone releasing factor, an interferon, a proinsulin, secretin, interleukin-2, a calcitonin or a hirudin.

**Claims for the following Contracting State : ES**

**1.** A process for the preparation of a genetically codable polypeptide, with coupling of the structural gene for this polypeptide in the correct reading frame via the gene for $\beta$-galactosidase, or for a fragment of $\beta$-galactosidase, to a regulator region, said $\beta$-galactosidase fragment coding for more than 250 amino acids, but significantly less than the complete $\beta$-galactosidase sequence, introduction of this gene structure into a bacterium, expression therein of an insoluble fusion protein, isolation thereof after cell disruption, and obtaining the desired polypeptide by chemical or enzymatic cleavage, which comprises codons for methionine and/or arginine and/or cysteine in the gene for $\beta$-galactosidase, or for the fragment of $\beta$-galactosidase, being replaced, in whole or in part, by codons of other amino acids.

**2.** The process as claimed in claim 1, wherein the $\beta$-galactosidase fragment is composed of a fusion of an amino-terminal and carboxyl-terminal part-sequence.

**3.** The process as claimed in claim 1 or 2, wherein the $\beta$-galactosidase fragment has about 300 to about 800 amino acids.

**4.** The process as claimed in one or more of the preceding claims, wherein the $\beta$-galactosidase fragment has about 320 to about 650 amino acids.

**5.** The process as claimed in one or more of the preceding claims, wherein the gene for the $\beta$-galactosidase fragment corresponds to a DNA sequence shown in Figure 1.

**6.** The process as claimed in one or more of the preceding claims, wherein the structural gene for the genetically codable polypeptide is coupled via an adaptor to the gene for the modified $\beta$-galactosidase or the $\beta$-galactosidase fragment.

**7.** The process as claimed in claim 6, wherein the adaptor codes for a poly(amino acid) sequence.

**8.** The process as claimed in one or more of the preceding claims, wherein a codon for an amino acid which allows chemical or enzymatic separation of the polypeptide from the $\beta$-galactosidase portion is located immediately upstream of the amino-terminal end of the structural gene.

**9.** The process as claimed in one or more of the preceding claims, wherein the genetically codable polypeptide is a derivative of growth hormone releasing factor, an interferon, a proinsulin, secretin, interleukin-2, a calcitonin or a hirudin.

**Revendications**
**Revendications pour les Etats contractants suivants : AT, BE, CH, DE, FR, GB, GR, IT, LI, LU, NL, SE**

**1.** Procédé pour la production d'un polypeptide génétiquement codable, dans lequel on combine à une région régulatrice le gène structural codant pour ce polypeptide, dans le cadre de lecture correct, par l'intermédiaire du gène codant pour la $\beta$-galactosidase ou pour un fragment de la $\beta$-galactosidase, ce fragment de $\beta$-galactosidase codant pour plus de 250 aminoacides mais nettement moins que la séquence totale de la $\beta$-galactosidase, on introduit ce gène structural dans une bactérie, dans laquelle est exprimée une protéine de fusion insoluble, on isole celle-ci après avoir ouvert la cellule et on obtient le polypeptide recherché par coupure chimique ou enzymatique, caractérisé en ce que, dans le gène codant pour la $\beta$-galactosidase ou pour le fragment de la $\beta$-galactosidase, des codons codant pour la méthonine et/ou l'arginine et/ou la cystéine sont remplacés en partie ou en totalité par des codons codant pour d'autres aminoacides.

**2.** Procédé selon la revendication 1, caractérisé en ce que le fragment de $\beta$-galactosidase consiste en une fusion d'une séquence partielle amino-terminale et d'une séquence partielle carboxy-terminale.

3. Procédé selon la revendication 1 ou 2, caractérisé en ce que le fragment de $\beta$-galactosidase comporte d'environ 300 à environ 800 aminoacides.

4. Procédé selon une ou plusieurs des revendications précédentes, caractérisé en ce que le fragment de $\beta$-galactosidase comporte d'environ 320 à environ 650 aminoacides.

5. Procédé selon la revendication 1, caractérisé en ce que, immédiatement avant l'extrémité amino-terminale du gène structural, se trouve un codon codant pour un aminoacide qui permet une séparation chimique ou enzymatique du polypeptide d'avec le fragment $\beta$-galactosidase.

6. Procédé selon une ou plusieurs des revendications précédentes, caractérisé en ce que le polypeptide génétiquement codable est un dérivé du facteur de libération d'hormone de croissance, un interféron, une proinsuline, la sécrétine, l'interleukine-2, une calcitonine ou une hirudine.

7. Structure génique, contenant une région régulatrice, un gène codant pour la $\beta$-galactosidase ou un fragment de $\beta$-galactosidase, ce fragment de $\beta$-galactosidase codant pour plus de 250 aminoacides mais nettement moins que la séquence totale de $\beta$-galactosidase, dans laquelle séquence de $\beta$-galactosidase des codons codant pour la méthionine et/ou l'arginine et/ou la cystine sont remplacés en partie ou en totalité par des codons codant pour d'autres aminoacides, et un gène structural codant pour un polypeptide génétiquement codable, qui est éventuellement soudé, par l'intermédiaire d'un segment de raccord, au gène codant pour la $\beta$-galactosidase modifiée ou le fragment de $\beta$-galactosidase, qui garantit le cadre de lecture correct.

8. Vecteur contenant une structure génique selon la revendication 7.

9. Bactérie, de préférence *E. coli,* contenant un vecteur selon la revendication 8.

10. Protéine de fusion, contenant de la $\beta$-galactosidase modifiée ou un fragment de $\beta$-galactosidase tel que défini dans une ou plusieurs des revendications 1 à 8, et un polypeptide d'eucaryote génétiquement codable, qui est de préférence un dérivé du facteur de libération d'hormone de croissance, un interféron, une proinsuline, la sécrétine, l'interleukine-2, une calcitonine ou une hirudine.

**Revendications pour l'Etat contractant suivant : ES**

1. Procédé pour la production d'un polypeptide génétiquement codable, dans lequel on combine à une région régulatrice le gène structural codant pour ce polypeptide, dans le cadre de lecture correct, par l'intermédiaire du gène codant pour la $\beta$-galactosidase ou pour un fragment de la $\beta$-galactosidase, ce fragment de $\beta$-galactosidase codant pour plus de 250 aminoacides mais nettement moins que la séquence totale de la $\beta$-galactosidase, on introduit ce gène structural dans une bactérie, dans laquelle est exprimée une protéine de fusion insoluble, on isole celle-ci après avoir ouvert la cellule et on obtient le polypeptide recherché par coupure chimique ou enzymatique, caractérisé en ce que, dans le gène codant pour la $\beta$-galactosidase ou pour le fragment de la $\beta$-galactosidase, des codons codant pour la méthonine et/ou l'arginine et/ou la cystéine sont remplacés en partie ou en totalité par des codons codant pour d'autres aminoacides.

2. Procédé selon la revendication 1, caractérisé en ce que le fragment de $\beta$-galactosidase consiste en une fusion d'une séquence partielle amino-terminale et d'une séquence partielle carboxy-terminale.

3. Procédé selon la revendication 1 ou 2, caractérisé en ce que le fragment de $\beta$-galactosidase comporte d'environ 300 à environ 800 aminoacides.

4. Procédé selon une ou plusieurs des revendications précédentes, caractérisé en ce que le fragment de $\beta$-galactosidase comporte d'environ 320 à environ 650 aminoacides.

5. Procédé selon une ou plusieurs des revendications précédentes, caractérisé en ce que le gène codant pour le fragment de $\beta$-galactosidase correspond à une séquence d'ADN selon la figure 1.

6. Procédé selon une ou plusieurs des revendications précédentes, caractérisé en ce que le gène structural codant pour le polypeptide génétiquement codable est soudé, par l'intermédiaire d'un segment de raccord, au gène codant pour la *β*-galactosidase modifiée ou le fragment de *β*-galactosidase.

7. Procédé selon la revendication 6, caractérisé en ce que le segment de raccord code pour une séquence de plusieurs aminoacides.

8. Procédé selon une ou plusieurs des revendications précédentes, caractérisé en ce que, immédiatement avant l'extrémité amino-terminale du gène structural, se trouve un codon codant pour un aminoacide qui permet une séparation chimique ou enzymatique du polypeptide d'avec le fragment *β*-galactosidase.

9. Procédé selon une ou plusieurs des revendications précédentes, caractérisé en ce que le polypeptide génétiquement codable est un dérivé du facteur de libération d'hormone de croissance, un interféron, une proinsuline, la sécrétine, l'interleukine-2, une calcitonine ou une hirudine.

**FIG.1**

FIG.2

FIG.3A

EcoRI

Amp$^r$

pBR 322

Tet$^r$

Ori

Pvu II

PvuII/EcoRI

EcoRI          Pvu II
Amp$^r$          Ori

EcoRI fill in

EcoRI$^-$          Pvu II
Amp$^r$          Ori

19

FIG.3B

## FIG. 3 C

BamHI ⎯⎯⎯⎯⎯ EcoRI  EcoRI⁻ EcoRI ⎯⎯⎯⎯⎯⎯⎯⎯⎯⎯⎯⎯⎯⎯⎯⎯ SmaI

P   O   RB   Ad   B   C   A

BamHI ⎯⎯⎯⎯⎯⎯⎯⎯⎯⎯ EcoRI ⎯⎯⎯⎯⎯⎯⎯⎯⎯⎯⎯⎯⎯ SmaI

P   O   RB  (Ad)   B   C   A

BamHI │ fill in

BamHI⁻ ⎯⎯⎯⎯⎯⎯⎯⎯⎯ EcoRI ⎯⎯⎯⎯⎯⎯⎯⎯⎯⎯⎯⎯⎯ SmaI

P   O   RB  (Ad)   B   C   A

EcoRI⁻ ⎯⎯⎯⎯⎯⎯⎯⎯⎯⎯⎯⎯ Pvu II

Amp^r      Ori

(pBR 322)

## FIG. 3D

EcoRI⁻/BamHI⁻

Amp^r

**pWI6**

Ori

P

O

RB   EcoRI

B

C

A

Pvu II⁻/ SmaI⁻

# FIG. 4

pWI6

EcoRI           EcoRI      EcoRI         EcoRI

B    C    A    Ori    Amp$^r$    P    O    RB      Pvu I

EcoRI
Pvu I
RB
P   O
EcoRI
B
Amp$^r$
C
pWZIP dMdC
A
Ori

FIG.5